# EUROPÄISCHE PATENTSCHRIFT

(11) **EP 1 806 113 B1**
(45) Veröffentlichungstag und Bekanntmachung des Hinweises auf die Patenterteilung: **07.08.2013**
(21) Anmeldenummer: 06000231.8
(22) Anmeldetag: 06.01.2006
(51) Int. Cl.: A61F 2/38, A61F 2/30

(54) **Verfahren zur Erstellung von individuellen Cranioplastiken und Cranioplastik**
Method of forming customized cranial implants and cranial implant
Procédé pour la fabrication d'un implant de crâne individuel et un implant de crâne

(43) Veröffentlichungstag der Anmeldung: 11.07.2007
(73) Patentinhaber: Lerch, Karl-Dieter, 57439 Attendorn (DE)
(72) Erfinder: Lerch, Karl-Dieter, 57439 Attendorn (DE)
(74) Vertreter: Kalkoff & Partner

(56) Entgegenhaltungen:
- DE-A1- 4 102 256
- DE-A1- 10 055 465
- DE-C1- 19 518 994
- US-A- 4 976 737
- US-A- 5 346 492

## Beschreibung

Cranioplastiken dienen dem Verschluß in der Schädeldecke erstellter Öffnungen für Eingriffe unter der Schädeldecke. In zurückliegender Zeit wurde für den Verschluß der für den Eingriff erstellten Öffnung in der Schädeldecke das hierfür aus der Schädeldecke herausgetrennte Knochenstück verwendet, das mit dem verbliebenen Schädelbein durch einen die Trennfuge zwischen verbliebenem Schädelbein und herausgetrenntem Knochenstück durchsetzenden, einen Ends mit einem über die Trennfuge zwischen verbliebenen Schädelbein und herausgetrenntem Knochenstück vorspringenden, von innen gegen das verbliebene Schädelbein und herausgetrennte Knochenstück zur Anlage kommenden Überstand versehenen Stift zusammengefaßt wurde, der gegenüber dem verbliebenen Schädelbein und dem herausgetrennten Knochenstück durch eine auf dem freien, über die Schädeldecke vorspringenden Ende des Stifts aufbringbare, von außen gegen das verbliebene Schädelbein und das herausgetrennte Knochenstück kraftschlüssig zur Anlage kommende Scheibe fixiert wird. (z. B. DE-196 03 887 C2 u. DE 296 149 21 U1). Für den nach dem operativen Eingriff vorgesehenen Verschluß der Öffnung in der Schädeldecke stand somit von vornherein das individuelle, beim Öffnen der Schädeldecke anfallende Paßstück zum Verschließen der erstellten Öffnung in der Schädeldecke nach dem Eingriff zur Verfügung. Diese Art und Weise des Verschlusses der für den operativen Eingriff in der Schädeldecke erstellten Öffnung nach dem operativen Eingriff kommt dann auch nach wie vor zur Anwendung. Vorgeschlagen worden ist dann auch schon, in der Schädeldecke gesetzte Bohrlöcher zum Ansetzen des die Trennfuge zwischen aus der Schädeldecke herauszutrennendem Knochenstück und verbleibendem Schädelbein legenden Trennwerkzeuges nach dem operativen Eingriff durch auf der Schädeldecke aufsitzende Abdeckplatten zu verschließen, die mittels Bohrungen im Uberstand der Abdeckplatten und diese Bohrungen durchsetzende Fixierungsmittel, dann auch selbstschneidende Schrauben, mit der Schädeldecke zusammengefaßt werden. (DE 41 11 856 C1). Diese Verschlüsse wären, auch wenn sie größer dimensioniert würden, völlig ungeeignet für den Verschluß der für den operativen Eingriff erstellten, in der Regel größer ausgelegten Öffnung in der Schädeldecke. Generell kommen in Zusammenhang von Fixierungsmitteln für die Zusammenfassung von aus der Schädeldecke herausgetrennten, nach dem operativen Eingriff wieder mit dem verbliebenen Schädelbein zusammenzufassenden Knochenstück sowie in Zusammenhang mit Bohrlöcher in der Schädeldecke verschließenden Abdeckplatten dann auch schon Titan- bzw. Titanlegierungen als körperverträgliche Werkstoffe zum Einsatz.

US-A- 4976 737 offenbart ein Verfahren zur Estellung von individuellen Cranioplastiken aus dauerfestem, körperverträglichen Material, wobei ein indivuduelles Mehrschichtenbild von einem Schädelknochen bereich und anschlieβend unter Answertung der ermittelten geometrischen Daten ein Modell dieses Schädel Knochen bereichs erstellt werden.

Der Erfinder hat sich die Aufgabe erstellt, ein Verfahren zu entwickeln, das es erleichtert, individuelle Cranioplastiken zu erstellen, gegebenenfalls dann auch die Möglichkeit eröffnet, die für die Erstellung der individuellen Cranioplastik ermittelten Daten zu speichern.

Die bei der Erfindung zugrundeliegende Aufgabe wird mit einem Verfahren zur Erstellung von individuellen Cranioplastiken aus dauerfestem, körperverträglichen Material gelöst, dass durch folgende Verfahrensschritte gekennzeichnet ist:

Erstellung eines individuellen Mehrschichtenbildes vom für einen operativen Eingriff aus der Schädeldecke zu entfernenden bzw. bereits entfernten Schädelknochenbereich,
Vermessung der geometrischen Daten des Schädelknochenbereichs anhand des erstellten Mehrschichtenbildes,
Erstellung eines Modells vom zu entfernenden bzw. entfernten Schädelknochenbereich unter Auswertung der ermittelten geometrischen Daten von diesem Bereich, Erstellung einer Formmulde mit Hilfe des zuvor erstellten Modells vom Schädelknochenbereich,
Erstellung der einen umlaufenden Überstand über den zu entfernenden bzw. entfernten Schädelknochenbereich aufweisenden Cranioplastik in der zuvor erstellten Formmulde,
beabstandetes Einbringen von Bohrungen im umlaufenden, sich am verbleibenden Schädelknochen abzustützenden Überstand der Cranioplastik für Fixierungselemente zur bleibenden Fixierung der Cranioplastik gegenüber der verbliebenen Schädeldecke.

Mit dem erfindungsgemäßen Verfahren ist gegenüber dem Stand der Technik der Vorteil verbunden, dass für das Verschließen der Schädeldecke aus der Schädeldecke herausgetrennte Stück der Schädeldecke nicht mehr auf das herausgetrennte Stück der Schädeldecke zurückgegriffen werden muß.

Die Unteransprüche 2 bis 5 heben ab auf Ausgestaltungen des erfindungsgemäßen Verfahrens.

Mit dem erfindungsgemäßen Verfahren zur Erstellung von individuellen Cranioplast ken ist also zum einen der Vorteil verbunden, dass exakt paßgerechte, insbesondere dann auch konturengerechte Cranioplastiken, gegebenenfalls dann auch im voraus, d.h. vor dem operativen Eingriff, erstellbar sind, verbunden ist damit der weitere Vorteil, daß die Erstellung der individuellen Cranioplastik kurzfristig auf schnellstemWege möglich wird, ein Vorteil, der sich insbesondere bei den hier in erster Line in Frage kommenden operativen Eingriffen im Bereich des menschlichen Gehirns positiv auswirkt.

Als Werkstoff für die Herstellung des für das Verfahren benötigten Schädelmodells bietet sich Acryl an. Das schließt andere geeignete Werkstoffe nicht aus.Für die Herstellung der Cranioplastik sowie der Cranioplastik am verbleibenden Schädelknochen feslegenden Schraubenelemente bietet sich insbesondere Titan an. Blech auf Titanbasis einer Wandstärke von 0,5 +/- 0,2 mm erweist sich dafür als hinreichend.

Die zum operativen Eingriff, damit dann auch zur Erstellung eines Modells von zu entfernenden bzw. entfernten Schädelknochenbereich benötigten geometrischen Daten des Mehrschichtbildes lassen sich vorteilhaft speichern

## Patentansprüche

1. Verfahren zur Erstellung von individuellen Cranioplastiken aus dauerfestem, körperverträglichen Material, **gekennzeichnet durch** folgende Verfahrensabschnitte:
Erstellung eines individuellen Mehrschichtenbildes vom für einen operativen Eingriff aus der Schädeldecke zu entfernenden bzw. bereits entfernten Schädelknochenbereich,
Vermessung der geometrischen Daten des Schädelknochenbereichs anhand des erstellten Mehrschichtenbildes,
Erstellung eines Modells vom zu entfernenden bzw. entfernten Schädelknochenbereich unter Auswertung der ermittelten geometrischen Daten von diesem Bereich,
Erstellung einer Formmulde mit Hilfe des zuvor erstellten Modells vom Schädelknochenbereich,
Erstellung der einen umlaufenden Überstand über den zu entfernenden bzw. entfernten Schädelknochenbereich aufweisenden Cranioplastik in der zuvor erstellten Formmulde,
beabstandetes Einbringen von Bohrungen im umlaufenden, sich am verbleibenden Schädelknochen abzustützenden Überstand der Cranioplastik für Fixierungselemente zur bleibenden Fixierung der Cranioplastik gegenüber der verbliebenen Schädeldecke.

2. Verfahren nach Anspruch 1, **gekennzeichnet durch** die Verwendung von Acryl zur Erstellung des Schädelmodells.

3. Verfahren nach Anspruch 1 oder 2, **gekennzeichnet durch** die Verwendung von Titan für die Erstellung der Cranioplastik sowie der Cranioplastik am verbleibenden Schädelknochen festlegenden Fixierungselemente

4. Verfahren nach einem der Ansprüche 1 bis 3 **gekennzeichnet durch** die Verwendung von Blech auf Titanbasis einer Wandstärke von 0,5 +/- 0,2 mm für die Erstellung der Cranioplastik.

5. Verfahren nach einem der Ansprüche 1 bis 4, **gekennzeichnet durch** eine der Erstellung des Modells vorausgehende Speicherung der geometrischen Daten des Schädelknochenbereichs des erstellten Mehrschichtenbildes.

## Claims

1. A method for creating customized cranial implants consisting of highly durable, physiologically compatible material, **characterized by** the following method steps:
creation of an individual multi-layer image from a cranial bone region to be removed or already removed from the skullcap for surgery,
measurement of the geometric data of the cranial bone region using the created multilayer image,
creation of a model of the removed or to be removed cranial bone region while evaluating the determined geometric data from this region,
creation of a mold with the assistance of the previously created model of the cranial bone region,
creation of the cranial implant having a peripheral protrusion above the removed or to be removed cranial bone region in the previously created mold,
spaced insertion of holes in the peripheral protrusion of the cranial implant abutting the remaining cranial bone for fixation elements to permanently affix the cranial implant to the remaining skullcap.

2. The method according to claim 1, **characterized by** the use of acrylic to create the skull model.

3. The method according to claim 1 or 2, **characterized by** the use of titanium to create the cranial implant, as well as the fixation elements affixing the cranial implant to the remaining cranial bone.

4. The method according to one of claims 1 to 3, **characterized by** the use of titanium-based sheet metal with a wall thickness of 0.5 ± 0.2 mm to create the cranial implant.

5. The method according to one of claims 1 to 4, **characterized by** saving the geometric data of the cranial bone region of the created multilayer image prior to creating the model.

## Revendications

1. Procédé pour la fabrication d'implants de crâne individuels en matériau résistant et biocompatible, **caractérisé par** les étapes de procédé suivantes :
réalisation d'une image multicouche individuelle de la zone osseuse crânienne, devant être enlevée, ou respectivement déjà enlevée de la calotte crânienne pour une intervention chirurgicale,
relevé des données géométriques de la zone osseuse crânienne à l'aide de l'image multicouche réalisée,
réalisation d'un modèle de la zone osseuse crânienne devant être enlevée ou respectivement déjà enlevée, accompagnée d'une analyse des données géométriques déterminées de cette zone,
réalisation d'un creux de moulage à l'aide du modèle de la zone osseuse crânienne réalisé précédemment,
réalisation, dans le creux de moulage réalisé auparavant, d'un implant de crâne présentant une saillie périphérique sur la zone osseuse crânienne à enlever ou respectivement enlevée,
mise en place, avec des intervalles, d'alésages dans la saillie périphérique de l'implant de crâne qui doit s'appuyer sur l'os crânien qui reste, pour des éléments de fixation en vue de la fixation permanente de l'implant de crâne par rapport à la calotte crânienne qui demeure.

2. Procédé selon la revendication 1, **caractérisé par** l'utilisation d'acrylique pour la réalisation du modèle de crâne.

3. Procédé selon la revendication 1 ou 2, **caractérisé par** l'utilisation de titane pour la réalisation de l'implant de crâne ainsi que des éléments de fixation fixant l'implant de crâne à l'os crânien qui reste.

4. Procédé selon une des revendications 1 à 3, **caractérisé par** l'utilisation de tôle à base de titane d'une épaisseur de paroi de 0,5 +/- 0,2 mm pour la réalisation de l'implant de crâne.

5. Procédé selon une des revendications 1 à 4, **caractérisé par** un enregistrement, précédant la réalisation du modèle, des données géométriques de la zone osseuse crânienne de l'image multicouche réalisée.
